# EUROPÄISCHE PATENTSCHRIFT

(11) **EP 3 265 162 B1**
(45) Veröffentlichungstag und Bekanntmachung des Hinweises auf die Patenterteilung: **14.12.2022**
(21) Anmeldenummer: 16712225.8
(22) Anmeldetag: 03.03.2016
(51) Int. Cl.: A61M 25/06, A61M 25/09

(54) **SICHERHEITSPUNKTIONSSYSTEM**
SAFETY PUNCTURING SYSTEM
SYSTÈME DE PONCTION SÉCURISÉ

(30) Priorität: 06.03.2015 DE 102015003026
(43) Veröffentlichungstag der Anmeldung: 10.01.2018
(73) Patentinhaber: Haindl, Hans, 30974 Wennigsen (DE)
(72) Erfinder: Haindl, Hans, 30974 Wennigsen (DE)
(74) Vertreter: Vossius & Partner Patentanwälte Rechtsanwälte mbB
(86) Internationale Anmeldenummer: PCT/EP2016/054576
(87) Internationale Veröffentlichungsnummer: WO 2016/142274

(56) Entgegenhaltungen:
- DE-A1- 4 434 169
- DE-A1-102012 104 058
- US-A1- 2002 045 843

## Beschreibung

Die vorliegende Erfindung betrifft ein Punktionssystem für die sichere Einführung von Führungsdrähten in Blutgefäße.

Nach regulatorischen Vorschriften in den USA sowie in Europa sollen bei risikoreichen Punktionen, zu denen Punktionen von Blutgefäßen grundsätzlich gehören, ausschließlich Punktionssysteme verwendet werden, die über Sicherheitsvorkehrungen zur Verhinderung von Nadelstichverletzungen verfügen. Allerdings gelten diese Forderungen nur dann, wenn solche Systeme für den jeweiligen Zweck auch verfügbar sind.

Inzwischen sind für zahlreiche Anwendungen in der Medizin derartige Sicherheitssysteme verfügbar, bisher allerdings nicht für das Einführen von Führungsdrähten bzw. Kathetern nach der sogenannten Seldinger-Technik. Die Seldinger-Technik ist eine besonders schonende Technik zur Einführung von Kathetern in Blutgefäße. Dabei wird mit einer relativ kleinen Kanüle das Blutgefäß punktiert und sodann ein flexibler Führungsdraht in das Blutgefäß eingeschoben. Danach wird die scharfe Kanüle entfernt und entweder direkt oder über die Zwischenschaltung einer sogenannten Schleuse ein Katheter in das Blutgefäß eingeführt. Dabei wird dieser Katheter von dem Führungsdraht, der sich bereits in dem Blutgefäß befindet, geführt. Nach Einführung des Katheters wird der Führungsdraht aus dem Blutgefäß herausgezogen. Für unterschiedliche Kanülen sind bereits diverse Sicherheitsvorrichtungen bekannt und auch in Gebrauch, die am Ansatz der Kanüle befestigt sind und seitlich auf die Kanüle geklappt werden können (vgl. beispielsweise US 2003/0229317 A1). Diese sind bei der Seldinger-Punktion aber schlecht zu verwenden, weil dabei häufig in der Tiefe liegende Gefäße punktiert werden und die Kanüle dabei in verschiedenste Richtungen bewegt wird, wobei derartige Sicherheitsvorrichtungen sich als störend erweisen. DE4434169A1 offenbart ein Punktionssystem mit Führungsdraht.

Der vorliegenden Erfindung liegt daher die Aufgabe zugrunde, eine Sicherheitsvorrichtung für ein Punktionssystem bereit zu stellen, das für die Seldinger-Technik geeignet ist. Diese Aufgabe wird durch ein Sicherheitspunktionssystem gemäß Anspruch 1 gelöst. Bevorzugte Ausführungsformen der Erfindung sind unter anderem in den abhängigen Ansprüchen beschrieben.

Dementsprechend betrifft die vorliegende Erfindung ein Sicherheitspunktionssystem mit einer Kanüle und einem Führungsdraht. Die Kanüle weist ein distales Ende mit einem Anschliff, ein proximales Ende und ein Lumen zur Aufnahme des Führungsdrahtes auf. Der Führungsdraht weist einen proximalen Abschnitt mit einem proximalen Ende und einem distalen Ende sowie einen distalen Abschnitt auf. Der proximale Abschnitt des Führungsdrahts ist lösbar mit dem distalen Abschnitt des Führungsdrahts verbunden. Bevorzugt weist das proximale Ende des proximalen Abschnitts des Führungsdrahts einen aufgeweiteten Bereich auf, der bevorzugt derart dimensioniert ist, dass er nicht in das Lumen der Kanüle eingeführt werden kann. Alternativ kann das proximale Ende des proximalen Abschnitts des Führungsdrahts einen Sperr-oder Blockadeabschnitt aufweisen, der auf alternative Art verhindert, dass dieser Abschnitt in das Lumen der Kanüle eingeführt werden kann. Dies kann beispielsweise mit Hilfe entsprechender Rastvorrichtungen und/oder Abschnitten mit entsprechend schlechten Gleitreibungseigenschaften bewerkstelligt werden.

Die Erfindung beruht unter anderem darauf, den üblicherweise einstückig ausgebildeten Führungsdraht zweiteilig auszubilden, wobei ein erster proximaler Abschnitt lösbar mit einem zweiten distalen Abschnitt verbunden ist. Der distale Abschnitt des Führungsdrahts wird entsprechend der Seldinger-Technik mit Hilfe der Kanüle in ein Blutgefäß eingeführt. Die Kanüle wird nun aus dem Blutgefäß entfernt und in proximaler Richtung zurückgezogen. Dabei verbleibt ein Teil des proximalen Abschnitts des Führungsdrahts im Lumen der Kanüle, da der aufgeweitete Bereich des proximalen Endes des proximalen Abschnitts nicht in das Lumen der Kanüle eingeführt werden kann. Damit nun der distale Abschnitt des Führungsdrahts für das Einführen eines Katheters genutzt werden kann, wird der proximale Abschnitt des Führungsdrahts vom distalen Abschnitt des Führungsdrahts gelöst, wobei bevorzugt das distale Ende des proximalen Abschnitts des Führungsdrahts distal über das distale Ende der Kanüle herausragt, so dass die Kanülenspitze bzw. der Anschliff der Kanüle durch dieses distale Ende gesichert wird.

Die vorliegende Erfindung erlaubt es demnach, die Kanüle eines herkömmlichen Seldinger-Systems äußerlich vollkommen unverändert zu lassen, so dass der Arzt seine Punktion wie bisher gewohnt durchführen kann. Das System besteht aus einer Seldinger-Punktionskanüle mit beliebigem Schliff, z. B. Cournand, die sich von außen bevorzugt durch nichts von einer üblichen Seldinger-Kanüle unterscheidet und die auch keinerlei Handhabungsänderung erfordert. Hat der Arzt dann das Blutgefäß getroffen, so führt er einen Führungsdraht ein, der sich auch von herkömmlichen Führungsdrähten an seinem distalen Ende überhaupt nicht unterscheidet und der, wie normalerweise üblich, in einem Dispenser verpackt sein kann. Dabei kann der Seldinger-Draht jede beliebige Spitzengestaltung haben, d.h. es kann sich um einen Seldinger-Draht mit gerader Spitze, mit J-Spitze oder leicht gekrümmter Spitze handeln. Auch besondere Konfigurationen, z. B. ein verlängerter weicher Abschnitt u. ä. sind genauso möglich wie bei jedem anderen Seldinger-Draht. Lediglich der proximale Abschnitt des Führungsdrahts, der die Funktionalität und Flexibilität des Systems in keiner Weise beeinträchtigt, weist zusätzliche strukturelle Merkmale auf. Den proximalen, lösbaren Abschnitt kann man sich dabei als eine Art Verlängerung aus Metall oder Kunststoff denken, die beispielsweise in einem kegelförmigen Endstück endet, über das die Kanüle nicht hinweg gezogen werden kann. Der Anwender zieht also die Kanüle über den Seldinger-Draht zurück, bis sie auf dieses Endstück rutscht; dann kann sie beispielsweise mittels einer speziell gestalteten Kupplung mitsamt dem Endstück vom Seldinger-Draht gelöst werden.

Der aufgeweitete Bereich bzw. der Sperr-oder Blockadeabschnitt ist vorzugsweise dazu geeignet, mit dem proximalen Ende der Kanüle in Eingriff zu treten, bevorzugt mit diesem zu verrasten. Die dabei entstehende Bindung ist bevorzugt unlösbar, d.h. der aufgeweitete Bereich und das proximale Ende können bevorzugt nicht zerstörungsfrei voneinander getrennt werden. Eine solche Verbindung kann beispielsweise dadurch bewerkstelligt werden, dass das proximale Ende der Kanüle einen Spritzenkegel mit einer Hinterschneidung aufweist und der aufgeweitete Bereich einen korrespondierenden Vorsprung oder eine korrespondierende Auskragung aufweist, der/die dazu geeignet ist, mit der Hinterschneidung, bevorzugt unlösbar, zu verrasten.

Es ist bevorzugt, dass der proximale Abschnitt des Führungsdrahts derart dimensioniert ist, dass das distale Ende des proximalen Abschnitts distal über das distale Ende der Kanüle herausragt, wenn der aufgeweitete Bereich bzw. der Sperr-oder Blockadebereich des Führungsdrahts an das proximale Ende der Kanüle angrenzt bzw. mit diesem in Eingriff tritt. Hierfür haben bevorzugt die Kanüle eine erste Länge und der proximale Abschnitt des Führungsdrahts eine zweite Länge, die größer ist als die erste Länge. Hierdurch wird sichergestellt, dass das distale Ende des proximalen Abschnitts des Führungsdrahts distal über das distale Ende der Kanüle mit dem Anschliff herausragt, nachdem die Kanüle aus dem Blutgefäß entfernt wurde und der proximale Abschnitt des Führungsdrahts vom distalen Abschnitt des Führungsdrahts wieder getrennt wurde. Der Anschliff der Kanüle wird dann zu diesem Zeitpunkt, d.h. nachdem die Kanülenspitze mit Blut in Berührung kam, durch das distale Ende des proximalen Abschnitts des Führungsdrahts gesichert, so dass ungewollte Verletzungen vermieden werden können.

Grundsätzlich können der proximale Abschnitt und der distale Abschnitt des Führungsdrahts auf unterschiedlichste Weise lösbar miteinander verbunden sein. Gemäß einer ersten bevorzugten Variante sind der proximale Abschnitt und der distale Abschnitt des Führungsdrahts über einen Bereich mit reduzierter Materialstärke und/oder eine Sollbruchstelle verbunden. Beispielsweise kann der Durchmesser des Führungsdrahts am Übergang zwischen proximalem und distalem Abschnitt so viel kleiner sein als der Durchmesser des restlichen Führungsdrahts, dass dieser Verbindungsabschnitt auf einfache Weise händisch durchbrochen werden kann. Bevorzugt ist der Durchmesser des Führungsdrahts im Verbindungsabschnitt kleiner als 50%, stärker bevorzugt kleiner als 40% besonders bevorzugt kleiner als 30% des restlichen Führungsdrahtdurchmessers. Alternativ kann die Sollbruchstelle auch mit Hilfe eines anderen Materials erreicht werden, das beispielsweise weniger reißfest und/oder bruchfest ist als das Material des restlichen Führungsdrahts. Die Sollbruchstelle kann auch durch eine Schweiß- und/oder Lötverbindung gebildet werden.

Gemäß einer weiteren bevorzugten Alternative können der proximale Abschnitt und der distale Abschnitt des Führungsdrahts mit Hilfe zweier Verbindungselemente bzw. Kupplungselemente lösbar verbunden sein. Bevorzugt weist das distale Ende des proximalen Abschnitts des Führungsdrahts ein erstes Verbindungselement auf und ein proximales Endes des distalen Abschnitts ein zweites Verbindungselement auf, das mit dem ersten Verbindungselement lösbar in Eingriff treten kann. Beispielsweise kann das erste oder zweite Verbindungselement einen verdünnten Halsabschnitt und einen abgerundeten, aufgeweiteten Abschnitt aufweisen und das jeweils andere Verbindungselement einen korrespondierenden Aufnahmeabschnitt mit einer aufweitbaren Öffnung aufweisen, in die der abgerundete, aufgeweitete Abschnitt eingeführt werden kann. Der abgerundete, aufgeweitete Abschnitt kann beispielsweise kugelförmig sein und der korrespondierende Aufnahmeabschnitt beispielsweise halbkugelförmig sein.

Alternativ kann das erste Verbindungselement mindestens zwei Federelemente mit radial nach innen vorstehenden Vorsprüngen und/oder Haken aufweisen, und das zweite Verbindungselement eine oder mehrere korrespondierende Aussparrung(en) und/oder Öffnung(en) aufweisen, in die die Vorsprünge und/oder Haken eingreifen können. Dabei federn die mindestens zwei Federelemente bevorzugt radial nach außen, wobei die Vorsprünge und/oder Haken in der entspannten Lage der Federn bevorzugt nicht mit den korrespondierenden Öffnungen bzw. Aussparungen in Eingriff stehen. Beispielsweise kann das distale Ende des proximalen Abschnitts des Führungsdrahts aus einem dünnwandigen Rohr bestehen, das an seinen distalen Ende mindestens zwei federnde, flache Zungen ausbildet, die an ihrem distalen Ende einwärts gerichtete Haken aufweisen. Die korrespondierende Aussparung am proximalen Ende des distalen Abschnitts des Führungsdrahts kann beispielsweise durch eine Drahtwendel gebildet werden, bei der die Ganghöhe größer ist als der Drahtdurchmesser.

Es ist ferner bevorzugt, dass eine verschiebbare Hülse im Bereich der Verbindung des proximalen Abschnitts des Führungsdrahts mit dem distalen Abschnitt des Führungsdrahts um den Führungsdraht herum angeordnet ist. Diese Hülse kann beispielsweise aus Kunststoff oder Metall bestehen und soll verhindern, dass die durch die Verbindungselemente gebildete Kupplung vorzeitig gelöst wird. Bevorzugt wird beim Zurückziehen der Kanüle über den Führungsdraht diese Hülse mitgenommen und verbleibt im Ansatz der Kanüle. Sobald die Kupplung bzw. die Verbindungselemente dann aus der Spitze der Kanüle austritt/austreten, kann/können sie problemlos gelöst werden.

Am distalen Ende des proximalen Abschnitts des Führungsdrahts kann ferner ein weiteres Schutzelement vorgesehen sein, wobei dieses weitere Schutzelement auch durch beispielsweise das erste Verbindungselement gebildet sein kann. Dieses weitere Schutzelement dient dazu, den Anschliff der Kanüle zumindest teilweise zu verdecken bzw. im Hinblick auf eine potentielle Verletzungsgefahr zu sichern. Dieses weitere Schutzelement kann beispielsweise ein kegelförmiger, bevorzugt elastischer, Kragen oder Vorsprung am distalen Ende des proximalen Abschnitts des Führungsdrahts sein. Wird die Kanüle so weit zurückgezogen, dass das distale Ende des proximalen Abschnitts des Führungsdrahts distal über das distale Ende der Kanüle herausragt, so kann sich dieser Kragen oder Vorsprung radial nach außen aufweiten und dementsprechend radial über den Anschliff der Kanüle hinausragen und diesen so bedecken. Anstelle eines Kragens oder Vorsprungs kann auch eine kegelförmige Drahtwendel vorgesehen sein, die sich beim Austritt aus der Kanülenspitze entspannt und entsprechend in radialer Richtung ausbreitet. Alternativ oder zusätzlich kann am distalen Ende des proximalen Abschnitts des Führungsdrahts ein rohrförmiger Abschnitt mit mehreren Längsschnitten vorgesehen sein, wobei die Rohrstreben zwischen den Längsschnitten wulstförmig aufgeweitet sind. Auch die oben bereits erwähnten Federelemente mit radial nach innen vorstehenden Vorsprüngen und/oder Haken können als weiteres Schutzelement dienen, wobei diese Federelemente gleichzeitig auch die Funktion eines Verbindungselements haben können.

Es ist bevorzugt, dass der aufgeweitete Bereich des Führungsdrahts dazu geeignet ist, mit dem proximalen Ende der Kanüle, bevorzugt unlösbar, in Eingriff zu treten, bevorzugt mit diesem zu verrasten, wobei das Schutzelement den Anschliff der Kanüle abdeckt, wenn der aufgeweitete Bereich des Führungsdrahts mit dem proximalen Ende der Kanüle in Eingriff tritt.

Gemäß einer bevorzugten Ausführungsform der vorliegenden Erfindung ist am proximalen Ende des distalen Abschnitts der Führungsdraht bzw. Kerndraht wenige Millimeter, bevorzugt 1-5 mm, vor dem proximalen Ende mit einem Verbindungselement in Form einer Außenhülle des Seldinger-Drahtes verschweißt. Diese Außenhülle überragt den Kerndraht um wenige Millimeter, bevorzugt 1-5 mm, und weist an ihrem Ende keine Kugel auf, sondern die Wendel ist halbkugelig eingezogen, so dass in der Mitte ein Loch verbleibt, das in etwa dem halben Innendurchmesser der Wendel entspricht. In diesem Loch sitzt ein vorzugsweise kugelförmiger Kopf eines geraden Verlängerungsstückes des Seldinger-Drahtes, das aus Metall oder aus Kunststoff sein kann und den proximalen Abschnitt des Führungsdrahtes bildet. Durch ein seitliches Abkippen dieses Verlängerungsstückes kann man es aus dem Ende des Seldinger-Drahtes herauslösen, so dass nach dem Ablösen dieses Verlängerungsdrahtes der Seldinger-Draht sich in seinen Handhabungseigenschaften praktisch nicht von jedem anderen Seldinger-Draht unterscheidet.

Alternativ kann das Verlängerungsstück angeschweißt und/oder angelötet sein, sich aber durch seitliches Abknicken leicht abbrechen lassen.

Der Ablauf ist nun so, dass mit der Kanüle punktiert wird und der Seldinger-Draht eingeführt wird. Danach wird die Kanüle über den Seldinger-Draht zurückgezogen, bis das gerade Verlängerungsende im Inneren der Kanüle liegt und dort mit einem geeigneten Mechanismus im Ansatz der Kanüle oder vor der Spitze der Kanüle verrastet. Der Ankopplungspunkt des Verlängerungsstückes liegt jetzt vor der Spitze der Kanüle, und die Kanüle lässt sich durch seitliches Abknicken mit diesem Verlängerungsstück vom Seldinger-Draht lösen. In diesem Zustand ragt das Ende der Verlängerung aus der Spitze der Kanüle heraus und schützt dadurch den Anwender davor, sich mit dem Kanülenschliff zu stechen. Wenn die Kanüle mit einem Hinterschliff versehen wird, ist auf diese Weise bereits vollständiger Schutz sichergestellt, weil dann die Spitze direkt an der Verlängerung des Seldinger-Drahtes anliegt.

Im Falle eines normalen Facettenschliffs, bei dem in diesem Zustand die Spitze der Kanüle etwas von der Verlängerung des Seldinger-Drahtes absteht, wird das Verletzungsrisiko ebenfalls vermindert. Die Verletzungssicherheit kann jedoch noch weiter verbessert werden, indem diese Verlängerung des Seldinger-Drahtes beispielsweise so konstruiert ist, dass sie vor der Spitze der Seldinger-Kanüle ihren Durchmesser vergrößert. Dies kann z.B. dadurch geschehen, dass ein Verlängerungsstück aus bevorzugt Kunststoff einen schirmähnlichen Kragen aufweist, der sich beim Hindurchziehen durch die Kanüle an diese Verlängerung anlegt und, sobald er aus der Spitze der Kanüle ausgetreten ist, sich wieder aufspreizt und damit die Kanülenspitze schützt. Eine weitere Möglichkeit besteht darin, dass die Verlängerung des Seldinger-Drahtes vor der Kanülenspitze liegend eine kleine Einschnürung aufweist, auf der sich eine schlüsselringähnliche Drahtwendel befindet. Diese Drahtwendel würde im konischen Ansatz der Kanüle zusammengedrückt und die Kanüle passieren können. Nach dem Austritt aus der Kanülenspitze würde sie wieder aufspringen, dann vor der Kanülenspitze liegen und diese damit schützen. Die Seldinger-Kanüle wäre damit, sobald sie vom Seldinger-Draht gelöst wird, an ihrer Spitze geschützt und könnte den Anwender oder andere nicht mehr verletzen. Damit die Verlängerung des Seldinger-Drahtes nicht zurückgleiten kann, kann sie entweder im Kanülenansatz automatisch verriegeln, oder ihr Zurückgleiten wird durch die vorstehend beschriebenen Mechanismen vor der Spitze verhindert.

Eine weitere Möglichkeit besteht darin, dass die Verlängerung aus einem Rohr besteht, das vor der Kanülenspitze Längsschnitte aufweist und die dazwischenliegenden Rohrwandungen federnd nach außen gewölbt sind.

Exemplarisch wird ein Verfahren zur verletzungssicheren Punktion eines Blutgefäßes unter Verwendung des erfindungsgemäßen Sicherheitspunktionssystems beschrieben. Hierfür wird ein Blutgefäß mit der Kanüle punktiert und anschließend der distale Abschnitt des Führungsdrahts in das Lumen der Kanüle eingeführt. Die Kanüle wird dann aus dem Blutgefäß entfernt und in proximaler Richtung soweit zurückgezogen, bis das distale Ende des proximalen Abschnitts des Führungsdrahts distal über das distale Ende der Kanüle herausragt. Anschließend wird der proximale Abschnitt vom distalen Abschnitt gelöst, wobei der proximale Abschnitt zumindest teilweise im Lumen der Kanüle verbleibt.

Die Erfindung wird nachfolgend unter Bezugnahme auf die Figuren näher beschrieben. Es zeigen:
- Fig. 1: eine Seitenansicht einer bevorzugten Ausführungsform des erfindungsgemäßen Punktionssystems;
- Fig. 2: eine Längsschnittansicht des Verbindungsabschnitts zwischen dem proximalen und dem distalen Abschnitt des Führungsdrahts gemäß einer bevorzugten Ausführungsform;
- Fig. 3: eine Seitenansicht der gesicherten Kanüle gemäß einer bevorzugten Ausführungsform;
- Fig. 4: eine Querschnittansicht des Verbindungsabschnitts zwischen dem proximalen Ende des Führungsdrahts und dem proximalen Ende der Kanüle gemäß einer bevorzugten Ausführungsform;
- Fig. 5: eine Querschnittansicht des Verbindungsabschnitts zwischen dem proximalen und distalen Abschnitt des Führungsdrahts gemäß einer bevorzugten Ausführungsform;
- Fig. 6 und 7: gesicherte Kanülenspitzen gemäß bevorzugter Ausführungsformen;
- Fig. 8: eine J-förmige distale Spitze des Führungsdrahtes;
- Fig. 9: eine Querschnittansicht des Verbindungsabschnitts zwischen dem proximalen und distalen Abschnitt des Führungsdrahts gemäß einer bevorzugten Ausführungsform;
- Fig. 10: eine gesicherte Kanüle und Einsatz des Schutzelements gemäß Figur 9;
- Fig. 11: eine Querschnittansicht des Verbindungsabschnitts zwischen dem proximalen und distalen Abschnitt des Führungsdrahts gemäß einer bevorzugten Ausführungsform;
- Fig. 12: eine gesicherte Kanüle und Einsatz des Schutzelements gemäß Figur 11;
- Fig. 13: eine Querschnittansicht des Verbindungsabschnitts zwischen dem proximalen und distalen Abschnitt des Führungsdrahts gemäß einer bevorzugten Ausführungsform; und
- Fig. 14: eine Querschnittansicht des Verbindungsabschnitts zwischen dem proximalen und distalen Abschnitt des Führungsdrahts gemäß einer bevorzugten Ausführungsform.

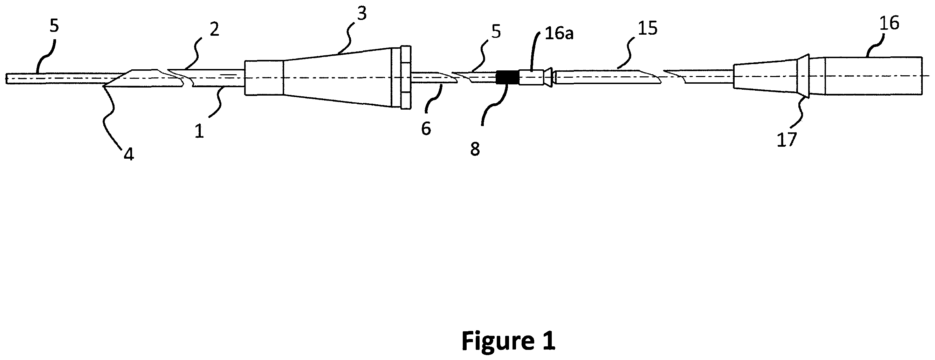

Die Figuren 1 bis 4 zeigen unterschiedliche Ansichten einer ersten bevorzugten Ausführungsform des erfindungsgemäßen Sicherheitspunktionssystems.

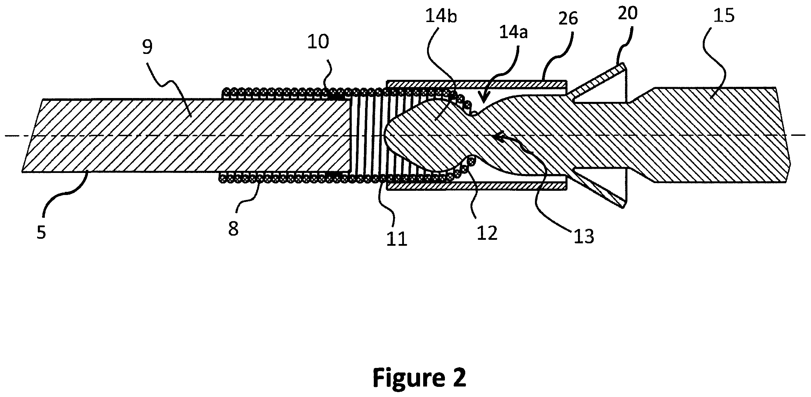

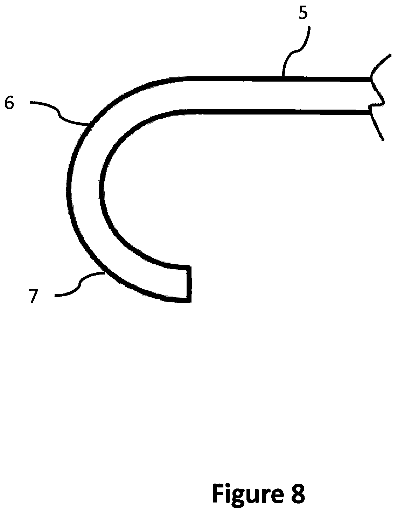

Das System besteht aus einer Kanüle 1 und einem Führungsdraht 5, der sich im Wesentlichen aus einem proximalen Abschnitt 15 mit einem proximalen Ende 16 und einem distalen Ende 16a sowie einem distalen Abschnitt 6 zusammensetzt, die lösbar miteinander verbunden sind. Die Kanüle 1 weist ein Kanülenrohr 2 und ein proximales Ende 3 mit einem Ansatz mit weiblichem Spritzenkegel auf, wobei das Kanülenrohr 2 an seiner distalen Spitze einen Anschliff 4 trägt. Dieser Anschliff kann für verschiedene Punktionen unterschiedlich ausgestaltet sein. Ebenso kann das distale Ende des distalen Abschnitts 6 des Führungsdrahts 5 sowohl gerade als auch als sogenannte J-Spitze 7 (vgl. Figur 8) ausgeführt sein. In der dargestellten Ausführungsform weist der Führungsdraht 5 einen Kerndraht 9 (vgl. Figur 2), der zur Regulierung der Flexibilität über seine Länge hin in unterschiedlichen Durchmessern ausgeführt sein kann, und eine Edelstahlwendel 8 auf. Der Kerndraht 9 ist an der Schweißstelle 10 mit der Wendel 8 verschweißt. Die Wendel 8 ragt an dem proximalen Ende 11 noch einige Millimeter, bevorzugt 1-5 mm, über das proximale Ende des Kerndrahtes 9 hinaus und ist in diesem Bereich hohl. Am äußersten Ende reduziert sich der Durchmesser der Wendel, so dass das Ende die Gestalt einer Halbkugel 12 einnimmt, die in ihrer Mitte eine Öffnung 13 hat. Diese Öffnung 13 ist elastisch aufdehnbar. In dieser Öffnung 13 steckt die kugelförmige Spitze 14b der FührungsdrahtVerlängerung 15, die den proximalen Abschnitt 15 bildet und die bevorzugt etwas länger ist als die Kanüle 1. Die Halbkugel 12 mit der Öffnung 13 einerseits und die kugelförmige Spitze 14b andererseits bilden so zwei korrespondierende Verbindungselemente, die lösbar miteinander in Eingriff treten können.

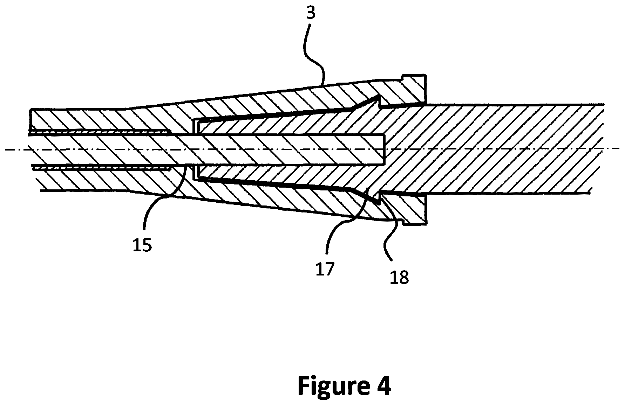

Der proximale Abschnitt 15 hat bevorzugt einen Durchmesser, der in etwa dem Durchmesser des distalen Abschnitts des Führungsdrahtes 5 entspricht und an seinem proximalen Ende ein beispielsweise kegeliges Endstück 16, das in den Spritzenkegel des Ansatzes 3 der Kanüle 1 passt. Der Kegel des Endstücks 16 kann Rasthaken oder Vorsprünge 17 tragen, die sich in entsprechenden Hinterschneidungen 18 im Spritzenkegel 3 unlösbar verriegeln können (vgl. Figur 4).

Der Führungsdraht 5 wird in einem Zustand, in dem der proximale und distale Abschnitt miteinander verbunden sind, wie dies in Figur 1 zu sehen ist, in das Lumen der Kanüle 1 eingeführt, nachdem diese ein Blutgefäß punktiert hat. Wenn die Kanüle 1 nach erfolgter Einführung des Führungsdrahtes 5 nun entfernt werden soll, kann sie nicht über dieses kegelige Endstück 16 hinweg gezogen werden. Stattdessen muss der ganze proximale Abschnitt 15 des Führungsdrahtes 5, auf dem die Kanüle 1 jetzt sitzt, vom distalen Abschnitt 6 des Führungsdrahts abgekoppelt bzw. getrennt werden. Hierzu dient die Verbindung bzw. Kupplung, die aus dem halbkugelförmigen Ende 12 des distalen Abschnitts 6 mit dem Loch 13 einerseits und der Kugel 14b des proximalen Abschnitts 15 andererseits besteht. Durch ein seitliches Abknicken löst sich die Kugel 14b aus der Öffnung 13, so dass sich der proximale und der distale Abschnitt voneinander lösen und die Kanüle 1 entfernt werden kann.

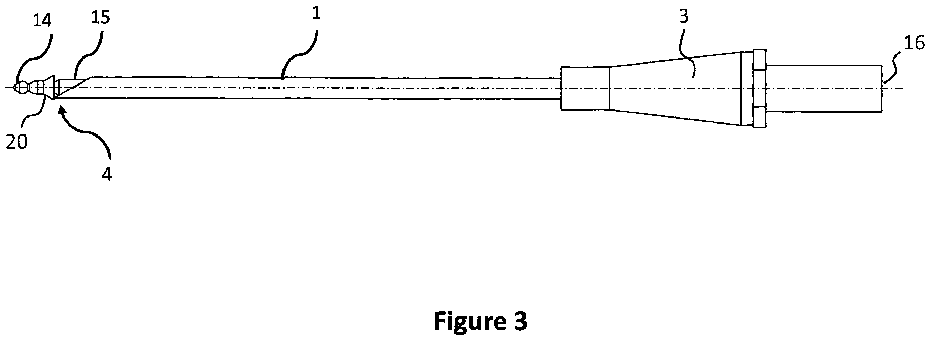

Bevorzugt wird der proximale Abschnitt bzw. die Verlängerung 15 des Führungsdrahtes 5 über geeignete Mittel im Inneren der Kanüle 1 verriegelt, so dass die Verlängerung 15 des Führungsdrahtes 5 über die Spitze der Kanüle hinaussteht und dadurch die Verletzungsgefahr durch die Kanülenspitze 4 reduziert wird (vgl. Figur 3). Diese Verriegelung kann beispielsweise im Ansatz 3 der Kanüle 1 erfolgen, indem dort eine Hinterschneidung 18 ausgebildet ist, in die ein federnder Steg 17 des kegeligen Endstückes 16 der Führungsdrahtverlängerung 15 einrastet (vgl. Figur 4).

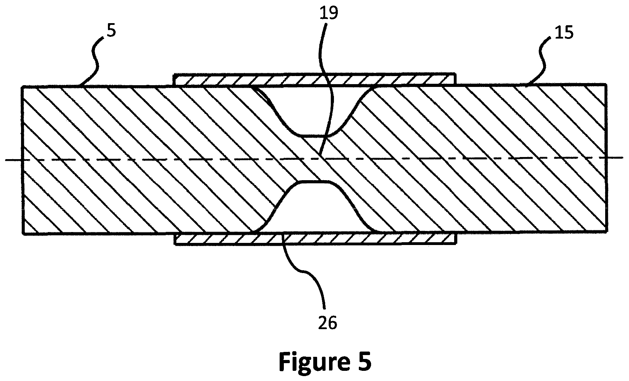

Alternativ können der proximale und distale Abschnitt des Führungsdrahts 5 durch eine Schweiß- und/oder Lötverbindung 19 miteinander verbunden sein (vgl. Figur 5), die sich durch Abknicken der Verlängerung vom Führungsdraht abbrechen lässt. In diesem Fall (wie auch bei der Ausführungsform der Figuren 1-4) kann eine kleine Hülse 26 aus Kunststoff oder Metall vorgesehen sein, um zu verhindern, dass, bevor die Kanüle in die geschützte Position zurückgezogen worden ist, die Verlängerung 15 des Führungsdrahtes 5 abgelöst werden kann. Diese Hülse ist bevorzugt verschieblich auf der Kupplung bzw. um den Verbindungsabschnitt zwischen proximalem und distalem Abschnitt herum angeordnet. Die Hülse ist bevorzugt so steif oder starr ausgeführt, dass ein Abknicken bzw. Abbrechen der Schweißstelle 19 bzw. der Kupplung gemäß den Figuren 1-4 wirksam verhindert wird. Beim Zurückziehen der Kanüle 1 über den Führungsdraht 5 wird diese Hülse 26 bevorzugt mitgenommen und verbleibt im Ansatz der Kanüle 1. Sobald die Kupplung dann aus der Spitze der Kanüle austritt, kann sie problemlos gelöst werden.

An den proximalen Abschnitt 15 des Führungsdrahts ist bevorzugt ein kegelförmiger, elastischer oder flexibler Kragen 20 angespritzt, der sich, wenn die Kanüle 1 darüber gezogen wird, an den Außenumfang des proximalen Abschnitts 15 anlegt, vorzugsweise an einen durchmesserreduzierten Bereich desselben, und sich, nachdem er die Spitze 4 der Kanüle 1 verlassen hat, wieder auffaltet (vgl. Figur 3). Dadurch steht dieser Kragen 20 einem Lösen der Kanüle 1 von dem proximalen Abschnitt entgegen, so dass auch auf das oben beschrieben Verrasten des federnden Stegs 17 mit der Hinterschneidung 18 verzichtet werden kann. Zudem schützt dieser Kragen zusätzlich die scharfe Kanülenspitze 4 bzw. deckte diese ab, so dass eine Verletzung noch unwahrscheinlicher wird.

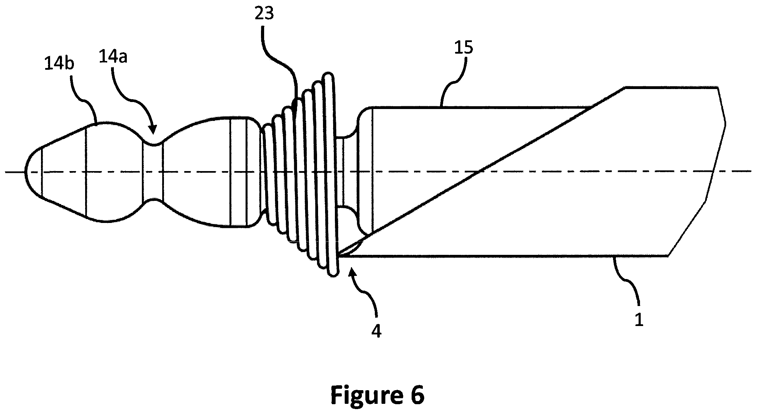

Anstelle dieses Schirmes 20 kann auch eine kleine, kegelige Drahtwendel 23 auf dem proximalen Abschnitt 15 des Führungsdrahtes 5 sitzen (vgl. Figur 6), die sich beim Überstreifen der Kanüle 1 ebenfalls an den Führungsdraht anlegen kann und danach vor der Spitze der Kanüle wieder aufgeht und sowohl ein Entfernen der Führungsdrahtverlängerung 15 verhindert als auch die Spitze 4 so abdeckt, dass eine Verletzung unwahrscheinlich wird.

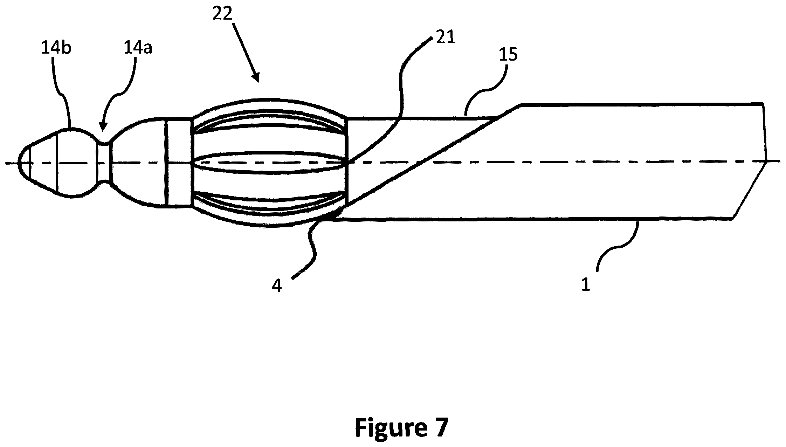

Eine weitere bevorzugte Ausführungsform dieses zusätzlichen Kanülenschutzes besteht darin, dass die Verlängerung 15 aus einem Rohr besteht, das kurz hinter der Kupplung an den Seldinger-Draht mehrere parallele, über den Umfang verteilte Längsschlitze 21 aufweist, die nach außen zu einem umlaufenden Wulst 22 gewölbt sind, aber federnd in die Ebene der Kanülenachse zurückbiegen können, so dass sie das Überstreifen der Kanüle 1 nicht behindern, danach aber vor der Kanülenspitze 2 aufgehen und diese vor Berührung schützen (vgl. Figur 7).

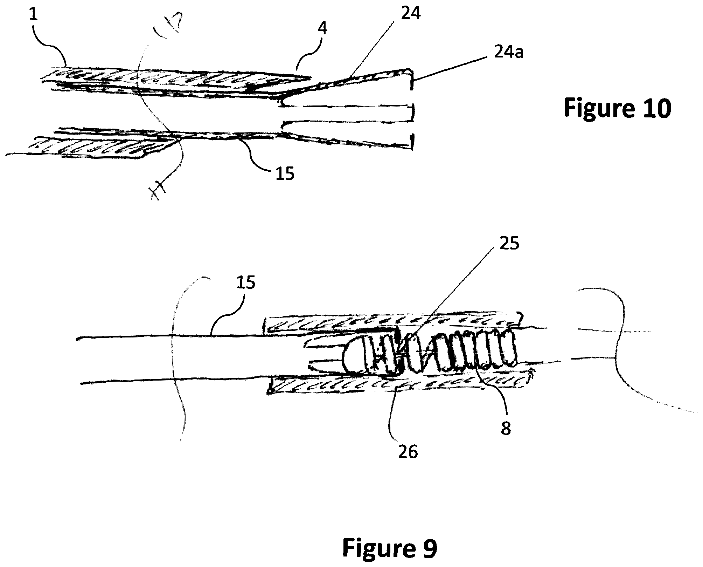

Eine weitere bevorzugte Ausführungsform dieses zusätzlichen Kanülenschutzes, die in den Figuren 9 und 10 zu sehen ist, besteht darin, dass ein erstes, am proximalen Abschnitt 15 des Führungsdrahts 5 vorgesehenes Verbindungselement mindestens zwei Federelemente 24 mit radial nach innen vorstehenden Vorsprüngen oder Haken 24a aufweist. Diese Vorsprünge oder Haken 24a greifen bevorzugt in eine oder mehrere Aussparung(en) und/oder Öffnung(en) 25 ein, die Teil eines zweiten, am proximalen Ende des distalen Abschnitts vorgesehenen Verbindungselements 8 sind (vgl. Figur 9). Dabei federn die mindestens zwei Federelemente 24 bevorzugt radial nach außen, wobei die Vorsprünge und/oder Haken 24a in der entspannten Lage der Federn bevorzugt nicht mit den korrespondierenden Öffnungen bzw. Aussparungen 25 in Eingriff stehen. In der hier dargestellten bevorzugten Ausführungsform werden die Aussparungen durch Freiräume 25 zwischen benachbarten, aber voneinander beabstandeten Windungen einer Drahtwendel 8 gebildet. Eine Hülse 26 verhindert eine radiale Bewegung der Federelemente 24 nach außen, so dass der proximale und distale Abschnitt des Führungsdrahts lösbar miteinander verbunden bleiben. Wird die Hülse 26 beim Zurückziehen der Kanüle 1 mitgenommen, können sich die Haken 24a frei nach außen bewegen und die Wendel 8 freigeben. Die Federelemente 24 verhindern dann in dem in Figur 10 dargestellten aufgeweiteten Zustand das Hineinziehen der Federelemente 24 in das Lumen der Kanüle 1, so dass der Anschliff 4 der Kanüle durch die Federelemente 24 gesichert wird. Mit anderen Worten wird auch bei dieser Ausführungsform das zusätzliche Schutzelement (vgl. Figur 10) durch das erste Verbindungselement (vgl. Figur 9) gebildet.

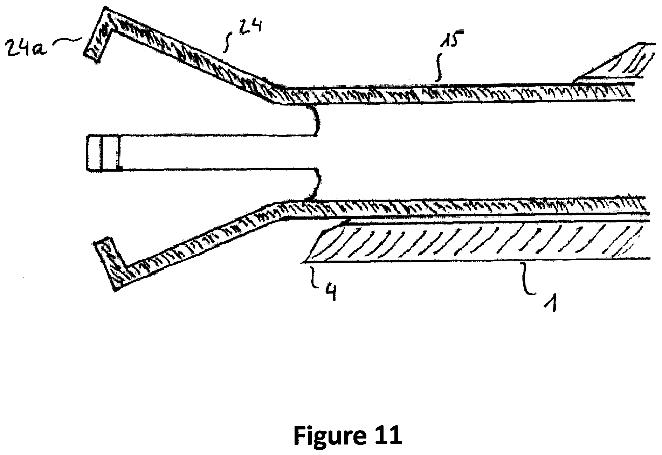

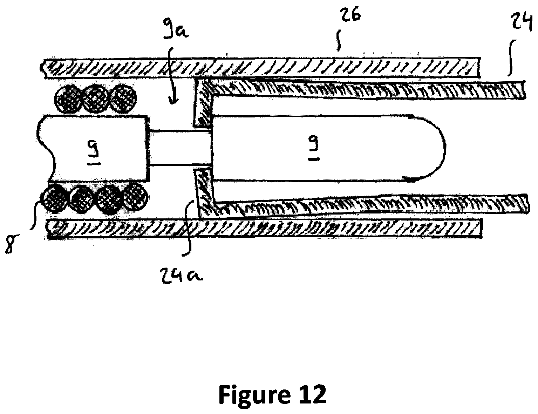

Eine ähnliche Ausführungsform ist in den Figuren 11 und 12 gezeigt, bei der ebenfalls das am proximalen Abschnitt 15 des Führungsdrahts 5 vorgesehene Verbindungselement mindestens zwei Federelemente 24 mit radial nach innen vorstehenden Vorsprüngen oder Haken 24a aufweist. Wie in Figur 12 zu sehen ist, können jedoch diese Vorsprünge bzw. Haken 24a nicht nur in Aussparungen eingreifen, die durch Freiräume 25 zwischen benachbarten, aber voneinander beabstandeten Windungen einer Drahtwendel 8 gebildet werden. Vielmehr interagieren bei dieser Ausführungsform die Vorsprünge bzw. Haken 24a mit einer hierfür vorgesehenen Aussparung bzw. Nut 9a im Kerndraht 9, der proximal über die Wendel 8 hinausragt.

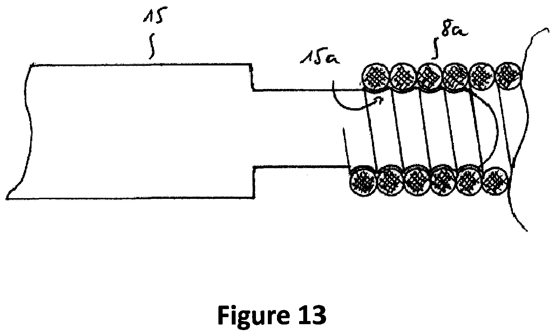

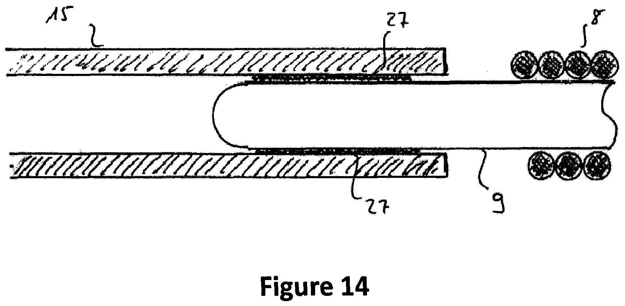

In den Figuren 13 und 14 sind schematisch zwei weitere Möglichkeiten zur lösbaren Verbindung des proximalen Abschnitts 15 des Führungsdrahts 5 mit dem Kerndraht 9 des Führungsdrahts 5 dargestellt. So kann beispielsweise der proximale Abschnitt 15 ein Außengewinde 15a aufweisen, das sich mit der Drahtwendel 8 (oder einem anderen Innengewinde) verschrauben lässt (vgl. Figur 13). Alternativ kann auch eine Verbindung mittels einer lösbaren Klebstoffverbindung wie z.B. einer Silikonschicht 27 erfolgen (vgl. Figur 14).

## Patentansprüche

1. Sicherheitspunktionssystem mit einer Kanüle (1) und einem Führungsdraht (5), wobei die Kanüle (1) ein distales Ende mit einem Anschliff (4), ein proximales Ende (3) und ein Lumen zur Aufnahme des Führungsdrahts (5) aufweist, wobei der Führungsdraht (5) einen proximalen Abschnitt (15) mit einem proximalen Ende (16) und einem distalen Ende (16a) und einen distalen Abschnitt (6) aufweist, wobei der proximale Abschnitt (15) lösbar mit dem distalen Abschnitt (6) verbunden ist und wobei das proximale Ende (16) des proximalen Abschnitts (15) einen aufgeweiteten Bereich aufweist **dadurch gekennzeichnet, dass** ferner die Kanüle (1) eine erste Länge hat und der proximale Abschnitt (15) des Führungsdrahts (5) eine zweite Länge hat, die größer ist als die erste Länge.

2. Sicherheitspunktionssystem nach Anspruch 1, wobei der aufgeweitete Bereich derart dimensioniert ist, dass er nicht in das Lumen der Kanüle (1) eingeführt werden kann.

3. Sicherheitspunktionssystem nach einem der Ansprüche 1 oder 2, wobei der aufgeweitete Bereich dazu geeignet ist, mit dem proximalen Ende (3) der Kanüle (1), bevorzugt unlösbar, in Eingriff zu treten, bevorzugt mit diesem zu verrasten.

4. Sicherheitspunktionssystem nach Anspruch 3, wobei das proximale Ende (3) der Kanüle (1) einen Spritzenkegel mit einer Hinterschneidung (18) aufweist und wobei der aufgeweitete Bereich einen Vorsprung (17) oder eine Auskragung aufweist, der/die dazu geeignet ist, mit der Hinterschneidung (18), bevorzugt unlösbar, zu verrasten.

5. Sicherheitspunktionssystem nach Anspruch 3 öder 4, wobei der proximale Abschnitt (15) des Führungsdrahts (5) derart dimensioniert ist, dass das distale Ende des proximalen Abschnitts (15) distal über das distale Ende der Kanüle herausragt, wenn der aufgeweitete Bereich des Führungsdrahts (5) mit dem proximalen Ende (3) der Kanüle (1) in Eingriff tritt.

6. Sicherheitspunktionssystem nach einem der vorigen Ansprüche, wobei der proximale Abschnitt (15) des Führungsdrahts (5) über einen Bereich mit reduzierter Materialstärke und/oder eine Sollbruchstelle (19) lösbar mit dem distalen Abschnitt (6) des Führungsdrahts verbunden ist.

7. Sicherheitspunktionssystem nach einem der vorigen Ansprüche, wobei der proximale Abschnitt (15) des Führungsdrahts (5) über eine Schweiß- und/oder Lötverbindung lösbar mit dem distalen Abschnitt (6) des Führungsdrahts verbunden ist.

8. Sicherheitspunktionssystem nach einem der vorigen Ansprüche, wobei der proximale Abschnitt (15) des Führungsdrahts (5) mit dem distalen Abschnitt (6) des Führungsdrahts mit Hilfe zweier Verbindungselemente lösbar verbunden ist.

9. Sicherheitspunktionssystem nach Anspruch 8, wobei das distale Ende (16a) des proximalen Abschnitts (15) des Führungsdrahts (5) ein erstes Verbindungselement (14; 24) aufweist und ein proximales Ende (11) des distalen Abschnitts (6) ein zweites Verbindungselement (8, 12) aufweist, das mit dem ersten Verbindungselement (14; 24) lösbar in Eingriff treten kann.

10. Sicherheitspunktionssystem nach Anspruch 9, wobei das erste Verbindungselement (14) einen verdünnten Halsabschnitt (14a) und einen abgerundeten, bevorzugt kugelförmigen, aufgeweiteten Abschnitt (14b) aufweist und/oder wobei das zweite Verbindungselement (8, 12) einen, bevorzugt halbkugelförmigen, Aufnahmeabschnitt (12) mit einer aufweitbaren Öffnung (13) aufweist; oderwobei das erste Verbindungselement (24) mindestens zwei Federelemente mit radial nach innen vorstehenden Vorsprüngen und/oder Haken (24a) aufweist und/oder wobei das zweite Verbindungselement (8) eine oder mehrere Aussparung(en) und/oder Öffnung(en) (25) aufweist, in die die Vorsprünge und/oder Haken eingreifen können.

11. Sicherheitspunktionssystem nach einem der vorigen Ansprüche, ferner mit einer verschiebbaren Hülse (26), die im Bereich der Verbindung des proximalen Abschnitts (15) des Führungsdrahts (5) mit dem distalen Abschnitt (6) des Führungsdrahts um den Führungsdraht (5) herum angeordnet ist.

12. Sicherheitspunktionssystem nach einem der vorigen Ansprüche, ferner mit einem weiteren Schutzelement (20; 21, 22; 23; 24) am distalen Ende des proximalen Abschnitts (15) des Führungsdrahts (5), wobei das Schutzelement (24) bevorzugt durch das erste Verbindungselement (24) gemäß Anspruch 12 gebildet wird.

13. Sicherheitspunktionssystem nach Anspruch 12, wobei das Schutzelement (20; 21, 22; 23; 24) eines oder eine Kombination der folgenden Elemente aufweist: kegelförmiger, bevorzugt elastischer, Kragen oder Vorsprung (20); bevorzugt kegelförmige Drahtwendel (23); rohrförmiger Abschnitt (22) mit mehreren Längsschnitten (21), wobei die Rohrstreben zwischen den Längsschnitten (21) wulstförmig aufgeweitet sind; Federelemente (24) mit radial nach innen vorstehenden Vorsprüngen und/oder Haken (24a).

14. Sicherheitspunktionssystem nach Anspruch 12 oder 13, wobei der aufgeweitete
Bereich des Führungsdrahts (5) dazu geeignet ist, mit dem proximalen Ende (3) der Kanüle (1), bevorzugt unlösbar, in Eingriff zu treten, bevorzugt mit diesem zu verrasten, und wobei das Schutzelement den Anschliff (4) der Kanüle (1) abdeckt, wenn der aufgeweitete Bereich des Führungsdrahts (5) mit dem proximalen Ende (3) der Kanüle (1) in Eingriff tritt.

## Claims

1. A safety puncturing system comprising a cannula (1) and a guidewire (5),
wherein the cannula (1) has a distal end having a bevel (4), a proximal end (3) and a lumen for receiving the guidewire (5), wherein the guidewire (5) has a proximal section (15) having a proximal end (16) and a distal end (16a) and a distal section (6), wherein the proximal section (15) is releasably connected to the distal section (6) and wherein the proximal end (16) of the proximal section (15) has an expanded region, **characterized in that**
the cannula (1) further has a first length and the proximal section (15) of the guidewire (5) has a second length greater than the first length.

2. The safety puncturing system according to claim 1, wherein the expanded region is dimensioned such that it cannot be introduced into the lumen of the cannula (1).

3. The safety puncturing system according to any of claim 1 or 2, wherein the expanded region is adapted to engage, preferably lock, with the proximal end (3) of the cannula (1), preferably in a non-releasable manner.

4. The safety puncturing system according to claim 3, wherein the proximal end (3) of the cannula (1) has a syringe cone having an undercut (18) and wherein the expanded region has a projection (17) or a collar which is adapted to lock with the undercut (18), preferably in a non-releasable manner.

5. The safety puncturing system according to claim 3 or 4, wherein the proximal section (15) of the guidewire (5) is dimensioned such that the distal end of the proximal section (15) projects distally beyond the distal end of the cannula when the expanded region of the guidewire (5) engages with the proximal end (3) of the cannula (1).

6. The safety puncturing system according to any of the preceding claims, wherein the proximal section (15) of the guidewire (5) is releasably connected to the distal section (6) of the guidewire in an area having a reduced material strength and/or a predetermined breaking point (19).

7. The safety puncturing system according to any of the preceding claims, wherein the proximal section (15) of the guidewire (5) is releasably connected to the distal section (6) of the guidewire by means of a welded and/or soldered joint.

8. The safety puncturing system according to any of the preceding claims, wherein the proximal section (15) of the guidewire (5) is releasably connected to the distal section (6) of the guidewire by means of two connecting elements.

9. The safety puncturing system according to claim 8, wherein the distal end (16a) of the proximal section (15) of the guidewire (5) has a first connecting element (14; 24) and a proximal end (11) of the distal section (6) has a second connecting element (8, 12) which can releasably engage with the first connecting element (14; 24).

10. The safety puncturing system according to claim 9, wherein the first connecting element (14) has a thinned throat section (14a) and a rounded, preferably spherical, expanded section (14b) and/or wherein the second connecting element (8, 12) has a preferably hemispherical receiving section (12) having an expandable opening (13); or
wherein the first connecting element (24) has at least two spring elements having radially inwardly projecting projections and/or hooks (24a) and/or wherein the second connecting element (8) has one or more recess(es) and/or opening(s) (25) with which the projections and/or hooks can engage.

11. The safety puncturing system according to any of the preceding claims, further comprising a movable sleeve (26) which is arranged around the guidewire (5) in the area of the connection of the proximal section (15) of the guidewire (5) to the distal section (6) of the guidewire.

12. The safety puncturing system according to any of the preceding claims, further comprising a further protecting element (20; 21, 22; 23; 24) at the distal end of the proximal section (15) of the guidewire (5), wherein the protecting element (24) is preferably formed by the first connecting element (24) according to claim 9.

13. The safety puncturing system according to claim 12, wherein the protecting element (20; 21, 22; 23; 24) comprises one or a combination of the following elements: conical, preferably elastic collar or projection (20); preferably conical wire helix (23); tubular section (22) having a plurality of longitudinal cuts (21), wherein the tube struts are expanded in a bulging manner between the longitudinal cuts (21); spring elements (24) having radially inwardly projecting projections and/or hooks (24a).

14. The safety puncturing system according to claim 12 or 13, wherein the expanded region of the guidewire (5) is adapted to engage, preferably lock, with the proximal end (3) of the cannula (1), preferably in a non-releasable manner, and wherein the protecting element covers the bevel (4) of the cannula (1) when the expanded region of the guidewire (5) engages with the proximal end (3) of the cannula (1).

## Revendications

1. Système de ponction de sécurité avec une canule (1) et un fil de guidage (5), dans lequel la canule (1) comporte une extrémité distale avec un biseau (4), une extrémité proximale (3) et une lumière pour recevoir le fil de guidage (5), dans lequel le fil de guidage (5) comporte une section proximale (15) avec une extrémité proximale (16) et une extrémité distale (16a) et une section distale (6), dans lequel la section proximale (15) est reliée de manière amovible à la section distale (6) et dans lequel l'extrémité proximale (16) de la section proximale (15) comporte un domaine évasé **caractérisé en ce que**, en outre, la canule (1) a une première longueur et la section proximale (15) du fil de guidage (5) a une deuxième longueur qui est plus grande que la première longueur.

2. Système de ponction de sécurité selon la revendication 1, dans lequel le domaine évasé est dimensionné de telle manière qu'il ne peut pas être introduit dans la lumière de la canule (1).

3. Système de ponction de sécurité selon l'une des revendications 1 ou 2, dans lequel le domaine évasé convient pour entrer en prise, de préférence de manière non amovible, avec l'extrémité proximale (3) de la canule (1), de préférence pour s'enclencher avec celle-ci.

4. Système de ponction de sécurité selon la revendication 3, dans lequel l'extrémité proximale (3) de la canule (1) comporte un cône de seringue avec une contre-dépouille (18) et dans lequel le domaine évasé comporte une saillie (17) ou une protubérance qui convient pour s'enclencher avec la contre-dépouille (18), de préférence de manière non amovible.

5. Système de ponction de sécurité selon la revendication 3 ou 4, dans lequel la section proximale (15) du fil de guidage (5) est dimensionnée de telle manière que l'extrémité distale de la section proximale (15) dépasse de manière distale au-delà de l'extrémité distale de la canule lorsque le domaine évasé du fil de guidage (5) entre en prise avec l'extrémité proximale (3) de la canule (1).

6. Système de ponction de sécurité selon l'une des revendications précédentes, dans lequel la section proximale (15) du fil de guidage (5) est reliée de manière amovible à la section distale (6) du fil de guidage par l'intermédiaire d'un domaine à épaisseur de matériau réduite et/ou d'un point destiné à la rupture (19).

7. Système de ponction de sécurité selon l'une des revendications précédentes, dans lequel la section proximale (15) du fil de guidage (5) est reliée de manière amovible à la section distale (6) du fil de guidage par l'intermédiaire d'une liaison soudée et/ou brasée.

8. Système de ponction de sécurité selon l'une des revendications précédentes, dans lequel la section proximale (15) du fil de guidage (5) est reliée de manière amovible à la section distale (6) du fil de guidage à l'aide de deux éléments de liaison.

9. Système de ponction de sécurité selon la revendication 8, dans lequel l'extrémité distale (16a) de la section proximale (15) du fil de guidage (5) comporte un premier élément de liaison (14; 24) et une extrémité proximale (11) de la section distale (6) comporte un deuxième élément de liaison (8, 12) qui peut entrer en prise de manière amovible avec le premier élément de liaison (14; 24).

10. Système de ponction de sécurité selon la revendication 9, dans lequel le premier élément de liaison (14) comporte une section de col (14a) amincie et une section (14b) évasée arrondie, de préférence sphérique, et/ou dans lequel le deuxième élément de liaison (8, 12) comporte une section de réception (12) de préférence hémisphérique avec une ouverture (13) qui peut être évasée; ou dans lequel le premier élément de liaison (24) comporte au moins deux éléments de ressort avec des saillies et/ou des crochets (24a) dépassant radialement vers l'intérieur et/ou dans lequel le deuxième élément de liaison (8) comporte un ou plusieurs évidements et/ou ouvertures (25) dans lesquelles les saillies et/ou les crochets peuvent pénétrer.

11. Système de ponction de sécurité selon l'une des revendications précédentes, en outre avec un manchon coulissant (26) qui est disposé autour du fil de guidage (5), dans le domaine de la liaison de la section proximale (15) du fil de guidage (5) avec la section distale (6) du fil de guidage.

12. Système de ponction de sécurité selon l'une des revendications précédentes, en outre avec un autre élément de protection (20; 21, 22; 23; 24) à l'extrémité distale de la section proximale (15) du fil de guidage (5), dans lequel l'élément de protection (24) est formé de préférence par le premier élément de liaison (24) selon la revendication 9.

13. Système de ponction de sécurité selon la revendication 12 dans lequel l'élément de protection (20; 21, 22; 23; 24) comporte un ou une combinaison des éléments suivants: une collerette ou saillie (20) conique, de préférence élastique; une hélice de fil de préférence conique (23); une section tubulaire (22) avec plusieurs entailles longitudinales (21), où les traverses tubulaires entre les entailles longitudinales (21) sont évasées en forme de bourrelet; des éléments de ressort (24) avec des saillies et/ou crochets (24a) dépassant radialement vers l'intérieur.

14. Système de ponction de sécurité selon la revendication 12 ou 13 dans lequel le domaine évasé du fil de guidage (5) convient pour entrer en prise avec l'extrémité proximale (3) de la canule (1), de préférence de manière non amovible, de préférence pour s'enclencher avec celle-ci, et dans lequel l'élément de protection recouvre le biseau (4) de la canule (1), lorsque le domaine évasé du fil de guidage (5) entre en prise avec l'extrémité proximale (3) de la canule (1).
